# EUROPEAN PATENT SPECIFICATION

(11) **EP 1 252 315 B1**
(45) Date of publication and mention of the grant of the patent: **21.06.2006**
(21) Application number: 01902408.2
(22) Date of filing: 05.02.2001
(51) Int. Cl.: C12N 15/16, C07K 14/59, A01K 67/027, C12P 21/00

(54) **METHOD FOR THE PRODUCTION OF A PROTEIN**
VERFAHREN ZUR HERSTELLUNG EINES PROTEINS
METHODE DE PRODUCTION D'UNE PROTEINE

(30) Priority: 04.02.2000 EP 00200395
(43) Date of publication of application: 30.10.2002
(73) Proprietor: Vermeiden Van der Linden Beheer B.V., 3633 AG Vreeland (NL)
(72) Inventor: VERMEIDEN, Jan, Pieter, Willem, NL-3633 AG Vreeland (NL)
(74) Representative: Wittop Koning, Tom Hugo
(86) International application number: PCT/EP2001/001217
(87) International publication number: WO 2001/057216

(56) References cited:
- EP-A- 0 404 458
- WO-A-99/35241
- US-A- 5 792 902
- GREENBERG N.M. ET AL.: "Expression of biologically active heterodimeric bovine follicle-stimulating hormone in milk of transgenic mice" PROCEEDINGS OF THE NATIONAL ACADEMY OF SCIENCES OF USA, vol. 88, October 1991 (1991-10), pages 8327-8331, XP002142421
- JOHN D.C.A. ET AL.: "Expression of an engineered form of recombinant procollagen in mouse milk" NATURE BIOTECHNOLOGY, vol. 17, April 1999 (1999-04), pages 385-389, XP002173716
- HOUDEBINE L.-M.: "Production of pharmaceutical proteins from transgenic animals" JOURNAL OF BIOTECHNOLOGY, vol. 34, 1994, pages 269-287, XP002929507 ISSN: 0168-1656
- SHARPE C.R. ET AL.: "Human apolipoproteins AI, AII, CII and CIII. cDNA sequences and mRNA abundance" NUCLEIC ACIDS RESEARCH, vol. 12, no. 9, 1984, pages 3917-3932, XP002173832
- FRAZER K.A. ET AL.: "The apolipoprotein(a) gene is regulated by sex hormones andacute-phase inducers in YAC transgenic mice" NATURE GENETICS, vol. 9, no. 4, April 1995 (1995-04), pages 424-431, XP001015585
- CHEN S.-H. ET AL.: "The complete cDNA and amino acid sequence of human Apolipoprotein B-100" THE JOURNAL OF BIOLOGICAL CHEMISTRY, vol. 261, no. 28, 5 October 1986 (1986-10-05), pages 12918-12921, XP001015573
- CALLOW M.J. ET AL.: "Expression of human apolipoprotein B and assembly of lipoprotein (a) in transgenic mice" PROCEEDINGS OF THE NATIONAL ACADEMY OF SCIENCES OF THE USA, vol. 91, March 1994 (1994-03), pages 2130-2134, XP002173833
- RAJENDRA KUMAR T. ET AL: 'Transgenic models to study Gonadotropin function: the role of Follicle-Stimulating Hormone in gonadal growth and tumorigenesis' MOLECULAR ENDOCRINOLOGY vol. 13, 1999, pages 851 - 865
- RAJENDRA KUMAR T.; LOW MALCOM J.: 'Gonadal steroid hormone regulation of human and mouse Follicle Stimulating Hormone beta-subunit gene expression in vivo' MOLECULAR ENDOCRINOLOGY vol. 7, 1993, pages 898 - 906
- MANN R.J. ET AL: 'Transgenic mice with chronically elevated Luteinizing Hormone are infertile due to anovulation, defects in uterine receptivity, and midgestation pregnancy failure' ENDOCRINOLOGY vol. 140, 1999, pages 2592 - 2601

## Description

The invention relates to a method for the production of a heteromeric protein, the protein comprising a first and a second peptide subunit, the second subunit being different from the first, the peptide subunits being associated to each other.

In the art, proteins of the above-mentioned type are known. For biological activity, association of the two different peptide subunits is a prerequisite. The first and second peptide subunits may originate from the same gene, e.g. in the case of insulin, but are usually encoded by different genes, such as is the case for antibodies and the protein hormones LH (lutenising hormone), FSH (follicle stimulating hormone), HCG (human chorion gonadopropine) and TSH (thyroid stimulating hormone). The latter all comprise an identical alpha chain and a hormone specific beta chain. It is to be understood that the protein according to the invention may comprise more than two different peptide subunits or several identical peptide subunits, as long as biological function of the protein requires association of two at least different peptide subunits. Such proteins are known as heteromeric proteins. In case the protein consists of two different peptide subunits, such a protein is also known as a heterodimeric protein. Further examples of such proteins are e.g. all other heterodimeric biological active proteins like hergulin, integrins, activins and inhibins.

In this specification, a "peptide" is defined as a single, optionally glycosylated, amino acid chain, of which the molecular weight is not defined. Such peptide will be capable to associate with at least one other peptide to form the heteromeric protein. The peptide preferably comprises an amino acid sequence of at least 15 amino acids.

In the art, the proteins of the above-mentioned type are either produced from unmanipulated animals, as is the case for HCG that is isolated from urine of pregnant human individuals, and LH and FSH, being isolated from the urine of postmenopausal females. Another way to produce such proteins is by genetic modified cells, wherein both the first and second peptide subunits are expressed, properly processed and associated to each other, forming the functional protein. At present, LH, FSH and HCG are prepared by genetically modified Chinese hamster ovarium cells (CHO).

EP-A-0 404 458 describes bacterial phage vectors comprising DNA-sequences encoding ovine FSH polypeptide subunits. It is proposed therein to express the subunits in a unicellular organism and to assemble these subunits subsequently in vitro.

However, the yield of genetically engineered proteins from cell lines or unicellular organisms is low, the isolation methods are difficult, resulting in a relative expensive protein product.

In the art, it is also known to produce genetically engineered proteins through transgenic animals. E.g. Nuijens, J.H. et al, J. Biol. Chem. 28:272 (13); 8802-7, 1997 describes the production of lactoferin from the milk of transgenic mice. The problem of the production of proteins by transgenic animals is the fact that the proteins, once expressed, may biologically be active in the animal and may result in undesired side-effects. Especially in the case of the production of protein hormones, e.g. those as described above, the transgenic animal may suffer from the expression of the said proteins. In genetic modified animals, producing a genetically engineered protein in the milk, about one percent of the expressed products appeared to "escape" from the milk into the body of the animal. In order to keep the transgenic animals in a healthy condition, high expression of the protein, i.e. of the peptides constituting with said protein, is therefore limited.

In order to obviate the above-mentioned drawbacks, the present invention relates to a method for the production of a heteromeric protein, the protein comprising a first and a second peptide subunit, the second chain being different from the first, the peptide subunits being associated to each other, comprising the following steps:
- bringing a first nucleic acid sequence, comprising
   - a first coding region, coding for the first peptide subunit, and
   - a first control region, operably linked to the first coding region, controlling the expression of the first peptide subunit in a first non human animal, into cells of the first non human animal and allowing expression of the first peptide subunit in the first animal by the said cells, the first peptide subunit being exogenous for the first animal,
   - isolating the first peptide subunit from the animal, or its offspring,
   - providing the second peptide subunit in isolated form,
   - forming the protein by bringing together the first peptide subunit isolated from the first animal with the second peptide subunit under conditions allowing association of both peptide subunits, thereby resulting in a biologically functional protein.

By the said method, only one of the first or second peptide subunits is produced by the animal, so that a functional protein cannot be formed, allowing high expression levels of the said peptide subunit. The peptide subunit is exogenous for the said animal; this means that the DNA coding for the said peptide subunit is a DNA molecule that has been introduced into the animal by a process such as transformation, transfection, electroporation, gene bombardment or any other method known in the art. It should be noted that it is possible that the host cell into which the said DNA-molecule has been inserted may itself also naturally harbour sequences encoding the same or a similar peptide, which is encoded by the natural genome of the said animal. For example, when a specific animal, e.g. a cow, is used in this invention, it is recognised that the said animal naturally contains, in its genome, one or more sequences encoding e.g. the alpha or beta chain of LH, FSH, CG or TSH, or the light and heavy antibody chains. A molecule such as this is referred to as an "endogenous" DNA-molecule, encoding an endogenous peptide.

The exogenous nucleic acid sequence, also referred to as the transgenic sequence, that is to be introduced into cells of a specific animal, e.g. a cow, is preferably not of cow origin. However the transgenic sequence may encode a peptide that is also endogenously produced by the animal.

The methods for bringing a nucleic acid sequence into an animal are known (see e.g. for a review Chan AWS, Cloning 1: 25-46, 1999), as well as the isolation of the transgenic products i.e. peptides, proteins (Niemann H et al, Transgenic Res. 8:237-47, 1999). According to the invention, at least one of the peptides of the (glyco) protein can thus be produced in a convenient manner with high yields. It is also possible, in case of the production of a protein, comprising more than two different peptide subunits, to have the more than one of the said peptide subunits produced by a single transgenic non-human animal, as long as at least one of the peptide subunits that are necessary for a functional protein product is not encoded on the transgenic nucleotide sequence, therewith avoiding any possible formation of a protein that is harmful to the animal. Preferably said nucleic acid sequence is stably introduced in the cells. This means that the transgenic nucleotide sequence is properly maintained, replicated and segregated during the cell cycle. Stable transformation may occur by chromosomal integration or by an extrachromosomal element, such as a suitable plasmid vector. Preferably the transgenic nucleotide sequence is integrated in the genome of the animal cells.

The invention relates in particular to the production of heteromeric proteins using transgenic non-human animals producing one or more peptides of the protein, wherein the expression of the protein as such would be harmful to the animals used, when the protein would be expressed to a level higher than the level of the natural counterpart of the said protein in the said animal. Among such proteins heteromeric enzymes and, in particular, the heteromeric protein hormones and antibodies are to be mentioned.

In case a single transgenic non-human animal is to produce more than one transgenic peptide, the sequence encoding said peptides may be located on a single nucleic acid, or on different transgenic sequences.

"Formation of the protein", as well as "reconstitution" as used herein refers to combining the different peptide subunits of a protein under conditions that allow proper association and folding of the peptides into the protein, preferably resulting in a biologically functional protein. It shall be clear that the protein is formed *in vitro* when the peptides are isolated from the animal before they are combined.

The skilled person will be aware of the fact that a transgenic peptide, produced according to the method of the present invention in a non-human animal may, despite the fact that the said peptide is exogenous for the said animal, associate with a second peptide, endogenously produced by the said animal. In case such association leads to a biological active hybrid protein, care has to be taken that such hybrid protein does not lead to the above-mentioned drawback within the transgenic animal.

The transgenic peptide, produced according to the method of the present invention may also be a mutein that is capable to associate with its natural or also mutated counterpart, leading a biologically functional heteromeric mutein.

The transgenic peptide or peptides can be isolated from the animal and be combined with the second peptide or the missing peptide(s) respectively to reconstitute the protein, resulting in a functional product. The second peptide subunit may e.g. be produced by tissue culture or be obtained from natural sources. However, it is highly preferred to have the second peptide subunit produced by a second transgenic non-human animal in a way as is described above. Thus in a preferred embodiment, the present invention relates to a method as described above, further comprising the following steps:
- bringing a second nucleic acid sequence, comprising
   - a second coding region, coding for the second peptide subunit, and
   - a second control region, operably linked to the second coding region, controlling the expression of the second peptide subunit in a second non human animal,
   into cells of the second non human animal, and allowing expression of the second peptide subunit in the second animal by the said cells, the second peptide subunit being exogenous for the second animal,
- isolating the second peptide subunit from the second animal, or its offspring, and
- forming the protein by bringing together the first peptide subunit isolated from the first animal or its offspring, with the second peptide subunit isolated from the second animal or its offspring under conditions allowing association of both peptide subunits, thereby resulting in a biologically functional protein.

In this way, both peptide subunits can be produced in high amounts, be isolated from the respective animals or their offspring, isolated and be brought together to reconstitute the protein.

It must be understood that the "first animal" and "second animal", as used herein, are different individuals; the first and second animals must not be of a different species, although this may be possible if desired (e.g. in view of the required glycosylation patterns that may be required for one or more of the transgenic peptide subunits).

It is preferred to at least partially purify the different peptides before the formation (reconstitution) of the protein by bringing together the said peptides. Purification of the peptides may lead to a better formation of the protein in vitro and to improved yields. Most preferably, both peptides are highly purified before the formation of the protein.

Preferably the nucleic acid sequences are brought into the non human animals in such a way that these sequences are propagated with the offspring of the said animals. In this specification, "offspring" means all animals, naturally bred or cloned, having the transgenic animal wherein the transgenic nucleic acid has originally been introduced as ancestor. The technique of producing transgenic animals that are capable of producing transgenic offspring is known in the art (Chan AWS, supra). Hereto, the transgenic sequences are incorporated in germ line cells of the transgenic non-human animal. This is highly advantageous, as in that use the transgenic sequences are propagated, and the production of the protein is not limited to a single individual transgenic animal. Therefore, the method of the invention also encompasses the production of the protein from peptides, isolated from the offspring of the non-human animal wherein the transgenic nucleic acid sequence has originally been introduced.

To obviate the problem of suffering of the transgenic animals from expressing proteins, that are harmful to the said animal, the first and second non-human animals can be mated, so that their offspring is capable of producing the heteromeric protein. A tissue or organ with which the protein to be expressed harmfully interacts, is removed from the said offspring. Thereto, such a method, as such not part of the invention as claimed, comprises the following steps:
- bringing a first nucleic acid sequence, comprising
   - a first coding region, coding for the first peptide subunit, and
   - a first control region, operably linked to the first coding region, controlling the expression of the first peptide subunit in a first non human animal,
   into at least germ line cells of the first non-human animal so that the first nucleic acid sequence is propagated with the offspring of the first animal,
- bringing a second nucleic acid sequence, comprising
   - a second coding region, coding for the second peptide subunit, and
   - a second control region, operably linked to the second coding region, controlling the expression of the second peptide subunit in a second animal,
   into at least germ line cells of the second non-human animal so that the second nucleic acid sequence is propagated with the offspring of the second animal,
   - producing at least one offspring animal by mating the first animal with the second animal, the said offspring animal expressing both peptide subunits and forming the protein,
   - isolating the thus formed protein from the said offspring animal,
wherein the protein formed in the offspring animal is harmful to the said animal by interacting with a distinct tissue or organ in the offspring animal, wherein the said tissue or organ is removed from the offspring animal.

In this case, the protein is formed *in vivo* and enables the production of transgenic animals that produce the complete transgenic heteromeric protein. The parent transgenic animals will substantially be healthy individuals, as the parent animals express one or more, but not all peptides, necessary for the functional protein. The parent lines can be bred without any drawbacks, whereas crossing both parent lines will lead to the offspring producing the desired protein, possibly resulting in limitation of their well-being to a certain extent. The offspring animals may however produce the desired protein in high levels, without the need for any reconstitution step. As such, a method wherein peptide chains of heteromeric proteins are expressed in different parent animals, which animals are mated so that the offspring expresses the heteromeric proteins is known from e.g. WO 99/35241 and from Greenberg et al., PNAS-USA (1991) 88, pp 8327-8331.

However, the desired protein may be harmful to the offspring animals in such extent that survival of the said offspring is not possible or unethical. In case the negative effect is caused by an interaction between the protein and a distinct tissue or organ of the offspring animal, the negative effect may be obviated by removing the said tissue or organ from the animal, if such removal still enables survival of the animal. The tissue or organ is preferably removed directly after birth or shortly thereafter. In particular, the production of gonadotropins such as LH, FSH and CG (chorion gonadotropine) can be produced when the gonads of the offspring animal are removed as such as not part of the invention. E.g, a female transgenic FSH producing offspring animal can be produced that is able to survive in a healthy state upon removal of the ovaries. Similarly, healthy TSH producing offspring animals can be producing upon removal of the thyroid gland.

In a preferred embodiment of the method according to the invention, the protein is purified after formation thereof in order to provide the protein product of high purity that can be used in e.g. medicaments.

In a preferred embodiment, at least the first peptide subunit is expressed by milk producing cells of a non-human mammal and excreted into the milk by the said mammal. When the transgenic peptide(s) is present in the milk, the said peptide can easily be obtained from the mammal by milking the mammal. When both peptides (and optionally additional peptides in case the protein consists more than two different peptides) are produced in milk of different non-human mammals, formation of the protein may take place by just mixing the milk under appropriate conditions and ratio. However, advanced methods may be used for proper protein formation; such methods are known in the art.

For expression of the required peptide subunit by milk producing cells of the transgenic non-human mammal, it is advantageous for the respective control region to comprise a casein promoter. The casein promoter has been proven to be very suitable for this purpose, although other regulatory sequences can also be used (Brink, MF et al, Theriogenolgy 53: 139-48, 2000; Whitelaw CB et al, Transgenic Res. 1: 3-13, 1991). For optimal expression, the control region preferably comprises controlling sequences, such as e.g. promoter, enhancer, ribosomal binding site etc., preferably derived from the same species as the respective transgenic animal. Thus it is preferred to incorporate a bovine casein promoter in the control region, when the peptide product is to be produced by a cow in her milk. However, it is very well possible to use a promoter of another mammalian species. It is also possible to use the bovine casein promoter in other species like mouse and rabbit.

In another preferred embodiment of the invention, the first coding region codes for the alpha chain of LH, FSH, HCG or TSH, and the second coding region for the corresponding beta chain of LH, FSH, HCG or TSH, respectively, or vice versa. By producing the alpha chain of the said peptide hormones in the one animal and the beta chain in the other, these peptide hormones can easily be produced in high quantities. Preferably the alpha chain and beta chain of the said hormones are of human origin to be optimally suitable for use in medicaments.

For veterinary medicaments, the peptide subunits of the protein preferably originate from the animal species for which the medicament is intended.

Preferably, at least one, preferably both, of the coding regions is accommodated in a genomic sequence. In numerous cases, proper expression of the peptides of the protein to be reconstituted require their natural genomic environment, such as intervening sequences (introns) and/or 3' or 5' untranslated sequences. It is for example shown that transgenic mice, containing cDNA for the complete FSH only produce a low amount of FSH (Greenberg et al., PNAS, USA (1991), 88, p. 8327/8331).

Preferably a nucleic acid vector is used, comprising:
- a coding region, coding for a first peptide chain of a protein, the protein comprising a first and a second peptide chain, the second chain being different from the first, the peptides being associated to each other, and
- a control region comprising a casein promoter, operably linked to the coding region, controlling the expression of the first peptide in an animal,
- the vector being void of a sequence resulting in the expression of the second peptide chain in the said animal.

Such a vector can be used to produce the transgenic non-human animal according to the invention. It has however to be avoided that the said vector results in expression of both first and second peptide chains, which may lead to the above-mentioned drawbacks of a transgenic animal producing high level of functional protein.

Said nucleic acid vector preferably comprises the coding region for the alpha or beta chain of LH, FSH, HCG or TSH, preferably from human origin. The vector can be a DNA or RNA vector as is known in the art. The coding region is preferably of genomic origin and preferably accommodated in a genomic sequence.

According to the invention non human animals can be obtained preferably a mammal, comprising in multiple cells, the first nucleic acid sequence according to the invention, the coding region thereof coding for an exogenous peptide. Preferably the nucleic acid sequence is propagated to the offspring of the said animal. By the presence of the said nucleic acid sequence, the exogenous peptide may be expressed in the animal and isolated for e.g. protein reconstitution purposes. In this specification, "animal" is to be understood as a non human higher animal, preferably a vertebrate, more preferably a mammal. Most preferred are mammals of which milk is easily isolated, i.e. cows, goats, sheep, rabbits etc.

The skilled person, aware of the above teaching will be capable of producing any protein such as a protein hormone, comprising different peptide chains that are needed for biological activity of the protein. Further, the choice of suitable animals can be easily assessed by the skilled person. In case the peptide to be produced needs glycosylation, an animal can be chosen that produces the said transgenic peptide with the required glycosylation pattern, or the glycosylation can be carried out in vitro after isolating the said peptide from the animal.

The invention will now be further explained in the following example, not being intended to limit the scope of the invention.

### EXAMPLES

The isolation, cloning and screening techniques used in the examples were done according common practise in the art; reference is made to Maniatis et al., Molecular cloning: A laboratory manual. Cold Spring Harbour Laboratory Press, Cold Spring Harbour, NY, USA.

### 1. Preparation of transgenic mice.

### a) Transgenic mice comprising transgenic DNA encoding the human alpha subunit of FSH

A mini-gene of the alpha subunit of human FSH is present between the *Bam*HI and *Bgl*II restriction sites (Matzuk and Boime, 1988, J. Cell Biol 106, 1049-1058). This fragment contains the fused exons 2 and 3 as a cDNA sequence, linked to the intron between exon 3 and 4, and exon 4, which contains 3'-sequences. This fragment was isolated after *Bam*HI/*Bgl*II digestion and ligated into a similarly cut transfer vector. Using site-directed mutagenesis and specific primers a *Cla*I site was added 5' of the *Bam*HI site and a NotI site was added 3' of the *Bgl*II site. From the resulting vector the mini-gene was cut by a *Cla*I/*Not*I digestion. The active fragment of the bovine alpha S1-casein gene promoter can be obtained as a 6.3 kb *Not*I*-Cla*I fragment (Bijvoet et al., Human Molecular Genetics, 7, 1815-1824, 1998). The two fragments were isolated and ligated, using a three-point ligation into the NotI-digested and dephosphorylated cosmid pWE15 (Bijvoet et al., Human Molecular Genetics, 7, 1815-1824, 1998). After excision with NotI, this results in a linear expression cassette ready for microinjection into the pronuclei of fertilised mouse oocytes, and the generation of mice transgenic for alpha-FSH. This vector system has been used earlier for the targeted expression of acid-glucosidase (Bijvoet et al., Human Molecular Genetics, 7, 1815-1824, 1998).

### b) Transgenic mice comprising transgenic DNA encoding the human beta subunit of FSH

A 3.7 kb fragment containing exon I, II, and III of the beta subunit of human FSH is present between the *Hin*dIII and *Bam*HI restriction sites in a genomic 16.5 kb insert. The 16.5 kb insert is present in a human genomic DNA library in the phage lMG3 (JL Keene et al., J. Biol. Chem. 264, 4769-4775, 1989). The exons in the 3.7 kb genomic sequence contain 5'-untranslated sequence, the entire coding region of beta FSH, and 1.1 kb of 3'-untranslated sequence.

The 3.7 kb fragment was isolated after *Hin*dIII/*Bam*HI digestion and ligated into a similarly cut transfer vector. Using site-directed mutagenesis and specific primers a *Cla*I site was added 5' of the *Hin*dIII site and a *Not*I site was added 3' of the *Bam*HI site. From the resulting vector the 3.7 kb fragment was cut by a *Cla*I/*Not*I digestion. The active fragment of the bovine alpha S1-casein gene promoter can be obtained as a 6.3 kb *Not*I-*Cla*I fragment (Bijvoet et al., Human Molecular Genetics, 7, 1815-1824, 1998). The two fragments were isolated and ligated, using a three-point ligation into the *Not*I-digested and dephosphorylated cosmid pWEl5 (Bijvoet et al., Human Molecular Genetics, 7, 1815-1824, 1998). After excision with *Not*I*,* this results in a linear expression cassette ready for microinjection into the pronuclei of fertilised mouse oocytes, and the generation of mice transgenic for beta FSH. This vector system has been used earlier for the targeted expression of acid-glucosidase (Bijvoet et al., Human Molecular Genetics, 7, 1815-1824, 1998).

The isolated DNA was micro-injected into the pronuclei of fertilised mouse oocytes (CBA/BrA X C57B16) and transferred to foster mothers using standard procedures (B. Hogan et al., Manipulating the mouse embryo: A laboratory manual. Cold Spring Harbour Laboratory Press, Cold Spring Harbour, NY, USA)

### 2. Presence and expression of transgenic sequences

DNA was extracted from tail biopsies, digested with *Eco*RI and subjected to agarose gel electrophoesis and Southern blotting to detect transgene. A *Nsi*I*-Nco*I bovine alpha S1-casei fragment was used as probe [Platenburg et al., Transgenic Res. 3, 99-108, 1994]. The copy number of the transgene was determined by Southern blot analysis on digestion with *Sac*I and hybridization with cDNA PCR fragments comprising sequences encoding the alpha or beta subunits as probes. The band intensities were compared with those of a serial dilution of plasmid DNA. A phosphoimager was used for quantification.

In total, twelve founders were obtained. Five with the alpha chain and 7 with the beta chain. They were crossed with wild-type CBA/Br X C57Bl/6 mice. The founders transmitted the transgene in a Mendelian fashion. Litter seizes were normal and littermates were indistinguishable. The copy of the transgene in the offspring ranged from 2 to 10 copies as judged from the intensity of the hybridisation signal on Southern blots from tail DNA using cDNA of the alpha chain, respectively of the beta chain.

Animals were killed by cervical dislocation. Tissue samples were embedded in tissue tek (Sakura Fine tek Europe BV) flash frozen and stored in liquid nitrogen for further analysis. Cryostat sections (6 micron) were incubated with rabbit anti-human FSH antiserum in combination with swine anti-rabbit IgG antibodies conjugated to horseradish peroxidase (Dako) and stained with diamonobenzidine (Dako). The sections were counterstained with Gill's heamatoxylin. Only sections of the lactating mammary glands were positive.

The rabbit anti human FSH was raised as follows. New Zealand white rabbits were weeky intracutaneously injected with human rFSH (Gonal F Seron) mixed with Freud adjuvant. After a statisfactory titer was reached the specifity of the antiserum was verified by an enzyme linked immunosorbent assay.

Total RNA was isolated from various tissues using the RNA-zolmethod. (RNazol: Campro Scientific). RNA (20 µg) was separated on a 1% agarose-formaldehyde gel and transferred Hybond-N membranes. The filter was hybridised with ³²P labelled alpha chain cDNA PCR fragments. Messenger RNA of the alpha chain was only detected in the RNA of the lactating mammary gland.

Analogous to the above, the presence of mRNA of the beta chain was determined. mRNA of the beta chain was only found in the RNA isolated from the lactating mammary gland.

### 3. Production of transgenic peptides in the mouse milk

Mouse milk was collected from day 7 after birth. Mouse milk was collected both from transgene and from non transgene animals (controls). Milk was flash frozen and stored at minus 20°C. The presence and the quantity of the alpha and the beta chain respectively in the milk of various mice was determined with an immuno-assay with a rabbit polyclonal antibody to FSH (supra). Mice expressing 2 mg/ml of the protein in their milk were used for further breeding.

No traces of the human alpha chain or the human beta chain could be detected in the milk of control animals.

### 4. Purification of the peptides from the milk.

Recombinant alpha chain was isolated from the milk. The initial purification procedure consisted of three steps. Milk fat and casein were removed by centrifugation. The whey fraction was loaded on a concanavalin A -Sepharose 4B column. Adsorbed proteins were eluted with 100 mM methyl alfa-D-mannonose pyranoside.

After concentration the proteins were loaded on a Sephadex G200 column. Samples of the eluate subjected to SDS-polyacryl-amide gel electrophoresis in the samples of the mice comprising the transgenic alpha chain, a strong band of 18 kD, the molecular weight of the transgenic product was observed.

The same procedure was followed to isolate the beta chain.

### 5. Reconstitution

The two fluids as obtained above, one comprising the alpha chain and one comprising the beta chain were mixed is a slightly reducing environment (6.4 mM cysteamine and 3.6 mM cystamine) at a pH of 8.7 at a final concentration of 0.02 mg/ml protein. Under these conditions the alpha and the beta chains recombine spontaneously (Huth JR et al, Endocrinology 135:911-918, 1994).

The fluid with the reconstituted recombinant hormone was concentrated. The concentrate was loaded on a Sephadex G200 column and fractionised. Samples of the eluate were subjected to SDS-polyacrylamide gel electrophoresis. A major band of 36 kD, corresponding to the molecular weight of the reconstituted transgenic product was observed.

### 6. Production of human FSH by offspring animals

Two strains of animals were established, one strain homozygote for the alpha chain of FSH and one strain for the beta chain of FSH. These strains were cross bred. The male offspring was not further used. The females were ovary ectomized in order to prevent disturbance of the well-being of the animal by hyperstimulation of the ovaries by leaking of the intact hormone into the blood. Milk production is induced in these adult females by hormone injections. The intact reconstituted hormone was present in the milk of these animals.

The recombinant hormone was purified from the milk as described in the above paragraph 4 and concentrated. The concentrate was loaded on a Sephadex G200 column and fractionised. Samples of the eluate were subjected to SDS-polyacrylamide gel electrophoresis. A major band of 36 kD, corresponding to the molecular weight of the transgenic protein product was observed.

### 7. Testing of the reconstituted protein with immuno-assay.

The 36 kD fraction of the reconstituted recombinant hormone was tested with a monoclonal antibody to human FSH (Delfia hFSH, Wallac). This antibody reacts with the beta chain of human FSH.

According to this assay, a specific activity of about 7.000-8.000 IU human FSH/mg protein was found to be present in the fluids.

### 8. Biological activity of the protein hormone

The biological activity was tested with a human granulosa cell assay (Foldesi I et al. Human Reprod 13:1455-60, 1998). Human granulosa cells were harvested at the time of follicular aspiration after ovarian hyperstimulation for *in vitro* fertilisation and cultured up to 9 days. After three pre-incubations days various doses of reconstituted recombinant hormone were added. Commercial rFSH (Gonal F, Serono the Hague), was used as standard. The addition of reconstituted recombinant hormone resulted in a dose- and time dependent increase in granulosa-lutein cell oestrogen production. The reconstituted recombinant hormone appeared to have an activity of about 8.000 IU FSH/mg protein.

## Claims

1. Method for the production of a heteromeric protein, the protein comprising a first and a second peptide subunit, the second subunit being different from the first, the peptide subunits being associated to each other, **characterised by** the following steps:
- bringing a first nucleic acid sequence, comprising
- a first coding region, coding for the first peptide subunit, and
- a first control region, operably linked to the first coding region, controlling the expression of the first peptide subunit in a first non human animal,
into cells of the first non human animal and allowing expression of the first peptide subunit in the first animal by the said cells, the first peptide subunit being exogenous for the first animal,
- isolating the first peptide subunit from the animal, or its offspring,
- providing the second peptide subunit in isolated form,
- forming the protein by bringing together the first peptide subunit isolated from the first animal with the second peptide subunit under conditions allowing association of both peptide subunits, thereby resulting in a biologically functional protein.

2. Method according to claim 1, **characterised in that** it comprises the following steps:
- bringing a second nucleic acid sequence, comprising
- a second coding region, coding for the second peptide subunit, and
- a second control region, operably linked to the second coding region, controlling the expression of the second peptide subunit in a second non human animal,
into cells of the second non human animal, and allowing expression of the second peptide subunit in the second animal by the said cells, the second peptide subunit being exogenous for the second animal,
- isolating the second peptide subunit from the second animal, or its offspring, and
- forming the protein by bringing together the first peptide subunit isolated from the first animal or its offspring, with the second peptide subunit isolated from the second animal or its offspring under conditions allowing association of both peptide subunits, thereby resulting in a biologically functional protein.

3. Method according to claim 1 or 2, **characterised in that** before formation of the protein the first and second peptides are at least partially purified.

4. Method according to any of the preceding claims, **characterised in that** the nucleic acid sequences are brought into the respective animals in such a way that the said sequences are propagated with the offspring of the said animals.

5. Method according to any of the preceding claims, **characterised in that** the protein is purified after formation thereof.

6. Method according to any of the preceding claims, **characterised in that** at least the first peptide subunit is expressed by milk producing cells of a mammal and excreted in the milk by the said mammal.

7. Method according to any of the preceding claims, **characterised in that** at least one of the first and second control region comprises a casein promoter.

8. Method according to claims 2-7, **characterised in that** the first coding region codes for the α- chain of LH, FSH, HCG or TSH, and the second coding region for the corresponding β- chain of LH, FSH, HCG or TSH, respectively, or vice versa, the α- chain and the β- chain preferably being human sequences.

9. Method according to any of the preceding claims, wherein at least one of the coding regions is accommodated in a genomic sequence.

## Patentansprüche

1. Verfahren zur Herstellung eines heteromeren Proteins, wobei das Protein eine erste und eine zweite Peptid-Untereinheit umfasst, wobei die zweite Untereinheit sich von der ersten unterscheidet, wobei die Peptid-Untereinheiten miteinander assoziiert sind, **ge-** k e n n z e i c h n e t durch die folgenden Schritte:
- Einbringen einer ersten Nukleinsäuresequenz, umfassend
- eine erste codierende Region, die für die erste Peptid-Untereinheit codiert, und
- eine erste Kontrollregion, die mit der ersten codierenden Region funktionsfähig verbunden ist, welche die Expression der erstem Peptid-Untereinheit in einem ersten nichtmenschlichen Tier kontrolliert,
in Zellen des ersten nichtmenschlichen Tieres und Gestattung der Expression der ersten Peptid-Untereinheit in dem ersten Tier durch die Zellen, wobei die erste Peptid-Untereinheit für das erste Tier exogen ist,
- Isolieren der ersten Peptid-Untereinheit aus dem Tier oder dessen Nachkommenschaft,
- Bereitstellen der zweiten Peptid-Untereinheit in isolierter Form,
- Bilden des Proteins durch Zusammenbringen der ersten Peptid-Untereinheit, die aus dem ersten Tier isoliert wurde, mit der zweiten Peptid-Untereinheit unter Bedingungen, welche die Assoziation beider Peptid-Untereinheiten erlauben, so dass ein biologisch funktionelles Protein resultiert.

2. Verfahren gemäß Anspruch 1, **dadurch gekennzeichnet, dass** es die folgenden Schritte umfasst:
- Einbringen einer zweiten Nukleinsäuresequenz, umfassend
- eine zweite codierende Region, die für die zweite Peptid-Untereinheit codiert, und
- eine zweite Kontrollregion, die funktionsfähig mit der zweiten codierenden Region verbunden ist, welche die Expression der zweiten Peptid-Untereinheit in einem zweiten nichtmenschlichen Tier kontrolliert,
in Zellen des zweiten nichtmenschlichen Tieres und Gestattung der Expression der zweiten Peptid-Untereinheit in dem zweiten Tier durch die Zellen, wobei die zweite Peptid-Untereinheit für das zweite Tier exogen ist,
- Isolieren der zweiten Peptid-Untereinheit aus dem zweiten Tier oder dessen Nachkommenschaft, und
- Bilden des Proteins durch Zusammenbringen der ersten Peptid-Untereinheit, die aus dem ersten Tier oder dessen Nachkommenschaft isoliert wurde, mit der zweiten Peptid-Untereinheit, die aus dem zweiten Tier oder dessen Nachkommenschaft isoliert wurde, unter Bedingungen, welche die Assoziation beider Peptid-Untereinheiten erlauben, so dass ein biologisch funktionelles Protein resultiert.

3. Verfahren gemäß Anspruch 1 oder 2, **dadurch gekennzeichnet, dass** vor der Bildung des Proteins die ersten und zweiten Peptide mindestens teilweise gereinigt werden.

4. Verfahren gemäß einem der vorhergehenden Ansprüche, **dadurch gekenn-** z e i c h n e t , dass die Nukleinsäuresequenzen in einer solchen Weise in die jeweiligen Tiere eingebracht werden, dass die Sequenzen mit der Nachkommenschaft der Tiere fortgepflanzt werden.

5. Verfahren gemäß einem der vorhergehenden Ansprüche, **dadurch gekenn- zeichnet**, dass das Protein nach dessen Bildung gereinigt wird.

6. Verfahren gemäß einem der vorhergehenden Ansprüche, **dadurch gekenn- zeichnet**, dass mindestens die erste Peptid-Untereinheit durch milcherzeugende Zellen eines Säugetiers exprimiert und durch das Säugetier in der Milch ausgeschieden wird.

7. Verfahren gemäß einem der vorhergehenden Ansprüche, **dadurch gekenn- zeichnet,** dass mindestens eine der ersten und zweiten Kontrollregionen einen Kaseinpromotor umfasst.

8. Verfahren gemäß Ansprüchen 2 bis 7, **dadurch gekennzeichnet, dass** die erste codierende Region für die α-Kette von LH, FSH, HCG oder TSH und die zweite codierende Region für die entsprechende β-Kette von LH, FSH, HCG bzw. TSH codiert oder umgekehrt, wobei die α-Kette und die β-Kette bevorzugt menschliche Sequenzen sind.

9. Verfahren gemäß einem der vorhergehenden Ansprüche, wobei mindestens eine der codierenden Regionen in einer genomischen Sequenz untergebracht ist.

## Revendications

1. Procédé pour la production d'une protéine hétéromère, la protéine comprenant des première et seconde sous-unités peptidiques, la seconde sous-unité étant différente de la première, les sous-unités peptidiques étant associées l'une à l'autre, **caractérisé par** les étapes consistant :
- à introduire une première séquence d'acide nucléique, comprenant
- une première région codante, codant pour la première sous-unité peptidique, et
- une première région de régulation, liée de manière fonctionnelle à la première région codante, régulant l'expression de la première sous-unité peptidique dans un premier animal non humain,
dans des cellules du premier animal non humain, et à laisser l'expression de la première sous-unité peptidique dans le premier animal s'effectuer par lesdites cellules, la première sous-unité peptidique étant exogène pour le premier animal,
- à isoler la première sous-unité peptidique de l'animal, ou de sa descendance,
- à fournir la seconde sous-unité peptidique sous forme isolée, et
- à former la protéine en réunissant la première sous-unité peptidique isolée du premier animal avec la seconde sous-unité peptidique dans des conditions permettant l'association des deux sous-unités peptidiques, avec ainsi pour résultat une protéine biologiquement fonctionnelle.

2. Procédé suivant la revendication 1, **caractérisé en ce qu'**il comprend les étapes consistant :
- à introduire une seconde séquence d'acide nucléique, comprenant
- une seconde région codante, codant pour la seconde sous-unité peptidique, et
- une seconde région de régulation, liée de manière fonctionnelle à la seconde région codante, régulant l'expression de la seconde sous-unité peptidique dans un second animal non humain,
dans des cellules du second animal non humain, et à laisser l'expression de la seconde sous-unité peptidique dans le second animal s'effectuer par lesdites cellules, la seconde sous-unité peptidique étant exogène pour le second animal, .
- à isoler la seconde sous-unité peptidique du second animal, ou de sa descendance, et
- à former la protéine en réunissant la première sous-unité peptidique isolée du premier animal ou de sa descendance avec la seconde sous-unité peptidique isolée du second animal ou de sa descendance dans des conditions permettant l'association des deux sous-unités peptidiques, avec ainsi pour résultat une protéine biologiquement fonctionnelle.

3. Procédé suivant la revendication 1 ou 2, **caractérisé en ce que**, avant la formation de la protéine, les premier et second peptides sont purifiés au moins partiellement.

4. Procédé suivant l'une quelconque des revendications précédentes, **caractérisé en ce que** les séquences d'acides nucléiques sont introduites dans les animaux respectifs de telle sorte que lesdites séquences soient multipliées avec la descendance desdits animaux.

5. Procédé suivant l'une quelconque des revendications précédentes, **caractérisé en ce que** la protéine est purifiée après sa formation.

6. Procédé suivant l'une quelconque des revendications précédentes, **caractérisé en ce qu'**au moins la première sous-unité peptidique est exprimée par les cellules productrices de lait d'un mammifère et est excrétée dans le lait par ledit mammifère.

7. Procédé suivant l'une quelconque des revendications précédentes, **caractérisé en ce qu'**au moins une des première et seconde régions de régulation comprend un promoteur de caséine.

8. Procédé suivant les revendications 2 à 7, **caractérisé en ce que** la première région codante code pour la chaîne α de la LH, de la FSH, de la HCG ou de la TSH et la seconde région codante pour la chaîne β correspondante de la LH, de la FSH, de la HCG ou de la TSH, respectivement, ou vice-versa, la chaîne α et la chaîne β étant de préférence des séquences humaines.

9. Procédé suivant l'une quelconque des revendications précédentes, dans lequel au moins une des régions codantes est logée dans une séquence génomique.
